# EUROPEAN PATENT SPECIFICATION

(11) **EP 2 671 514 B1**
(45) Date of publication and mention of the grant of the patent: **17.12.2014**
(21) Application number: 13170881.0
(22) Date of filing: 06.06.2013
(51) Int. Cl.: A61B 8/12, A61B 1/00, A61B 8/08

(54) **Ultrasonic endoscope**
Ultraschallendoskop
Endoscope à ultrasons

(30) Priority: 07.06.2012 JP 2012129900
(43) Date of publication of application: 11.12.2013
(73) Proprietor: Fujifilm Corporation, Minato-ku Tokyo 106-8620 (JP)
(72) Inventor: Morimoto, Yashuhiko, Kanagawa, 258-8538 (JP)
(74) Representative: Klunker . Schmitt-Nilson . Hirsch

(56) References cited:
- JP-A- H05 344 973
- JP-A- 2007 215 634
- US-B1- 6 238 336
- US-B1- 6 338 717

## Description

### BACKGROUND OF THE INVENTION

### 1. Field of the Invention

The present invention relates to an ultrasonic endoscope, and particularly, to an ultrasonic endoscope including an ultrasonic observation portion that has an ultrasonic transducer at a tip part of an insertion section to be inserted into a body cavity, and a treatment tool lead-out portion (treatment tool lead-out port) that has an erecting base capable of changing the lead-out direction of the treatment tool from the treatment tool channel.

### 2. Description of the Related Art

As ultrasonic endoscopes, an ultrasonic endoscope including an electronic scanning type ultrasonic transducer on a tip side of an insertion section of an endoscope is known. While an ultrasonic image of a lesion is acquired by the ultrasonic transducer, puncturing the lesion with a puncturing treatment tool led out into a body cavity through a treatment tool channel so as to collect cells or the like is performed.

In this type of ultrasonic endoscope, an endoscopic observation portion for acquiring an optical observation image (referred to as endoscopic image in the present specification), an ultrasonic observation portion for acquiring an ultrasonic image (tomographic image), and a treatment tool lead-out portion for leading out a treatment tool inserted through a treatment tool channel into the body cavity are arranged at a tip part (tip rigid part) of an insertion section to be inserted into the body cavity.

In a related-art ultrasonic endoscope, as in JP1993-344973A (JP-H5-344973), an ultrasonic transducer of the ultrasonic observation portion is arranged at the tip part, and the treatment tool lead-out portion (treatment tool lead-out port) is arranged on a base end side of the ultrasonic transducer. An observation optical system of the endoscopic observation portion is arranged at one side portion of the treatment tool lead-out port.

The treatment tool lead-out port is provided with an erecting base (referred to as a raising base) capable of changing the lead-out direction of a treatment tool led out of a conduit opening (conduit lead-out port) of the treatment tool channel. The erecting base bends a treatment tool from the treatment tool channel, which is led out of the conduit opening of the treatment tool channel, by a guide surface so as to guide the treatment tool in a predetermined lead-out direction, and is rotated around a rotational axis orthogonal to a scanning plane of the ultrasonic transducer so as to change the lead-out direction.

The treatment tool lead-out port including such an erecting base is configured so as to lead out the treatment tool from a position on almost the same plane as a scanning plane that is scanned with an ultrasonic wave by the ultrasonic transducer and so as to lead out the treatment tool parallel to the scanning plane irrespective of the erection angle of the erecting base. Accordingly, the treatment tool led out of the treatment tool lead-out portion advances along the scanning plane, and is projected on an ultrasonic image acquired by the ultrasonic observation portion.

The following ultrasonic endoscopes are suggested in JP2007-215634A and JP2010-4945A with respect to the ultrasonic endoscope of such a general configuration of the related-art.

JP2007-215634A suggests an ultrasonic endoscope including a mechanism that changes the direction of the rotational axis for turning the erecting base to change the erection angle thereof, to a direction around an axis orthogonal to the rotational axis. According to this, by changing the direction of the rotational axis of the erecting base to a direction different from the direction orthogonal to the scanning plane, the lead-out direction of the treatment tool from the treatment tool channel can be adjusted even in a direction different from a direction parallel to the scanning plane, and the treatment tool can be precisely guided even to a treatment region that is not on the scanning plane.

JP2010-4945A suggests an ultrasonic endoscope that enables an endoscope to be inserted into a body cavity so as to perform an endoscopic retrograde cholangiopancreatography (ERCP) and in a case where a suspected lesion, such as cancer, is found, enables endoscopic ultrasound-guided fine-needle aspiration (FNA) to be performed without extracting the endoscope out of the body cavity so as to collect tissues of the lesion to perform a pathological examination.

According to this, the insertion section is provided with a first treatment tool channel and a second treatment tool channel, and a side-view-type endoscopic observation portion for acquiring an endoscopic image, and a first erecting base that erects a treatment tool, such as an imaging tube led out of a lead-out port of the first treatment tool channel, within a visual field of the endoscopic observation portion that is disposed on the tip side of the tip rigid part. Thereby, ERCP can be performed using the first treatment tool channel, the first erecting base, and the endoscopic observation portion.

On the other hand, an ultrasonic transducer for acquiring an ultrasonic image, and a second erecting base that erects a treatment tool, such as a puncture needle led out of a lead-out port of the second treatment tool channel, within an observation range (scanning region) of the ultrasonic transducer are disposed on the base end side of the rigid tip part. This makes it possible to perform FNA using the second treatment tool channel, the second erecting base, and the ultrasonic transducer without extracting the endoscope out of the body cavity after ERCP.

Additionally, the endoscope of this JP2010-4945A discloses that the first erecting base arranged further toward the tip side than the ultrasonic transducer may be made rotatable around an axis along an insertion axis of the insertion section, and the treatment tool led out of the lead-out port of the first treatment tool channel may be made to tilt within the scanning region of the ultrasonic transducer so that the treatment tool can also be observed by the ultrasonic image.

### SUMMARY OF THE INVENTION

Incidentally, as in the tip part of the ultrasonic endoscope described in JP1993-344973A (JP-H5-344973), in the related-art, the erecting base of the treatment tool lead-out port leads out the treatment tool from the treatment tool channel from a position on the scanning plane of the ultrasonic transducer. Therefore, a substantial center of the treatment tool lead-out port and the erecting base in the width direction (direction orthogonal to the scanning plane) is arranged so as to be located at a position on the scanning plane. Since the observation optical system of the endoscopic observation portion is arranged at one side portion of the treatment tool lead-out port, a non-used region that is not effectively used is present at the other side portion. Accordingly, the diameter of the tip part can be reduced by reducing the non-used region. In that case, however, it is necessary to arrange the treatment tool lead-out port (erecting base) so as to shift to the non-used region side, and to arrange the observation optical system so as to shift to the scanning plane side correspondingly.

However, if the treatment tool lead-out port (erecting base) is arranged so as to shift to the non-used region side, the treatment tool from the treatment tool channel is no longer led out of a position on the scanning plane. Therefore the treatment tool may not pass through the scanning region of the ultrasonic transducer over a wide range. Accordingly, the treatment tool lead-out port cannot be simply arranged so as to shift to the non-used region side.

On the other hand, in a case where the treatment tool lead-out port is arranged so as to shift to the non-used region side to reduce the diameter of the tip part, it is considered that the treatment tool is configured so as to pass through the scanning region of the ultrasonic transducer by obliquely tilting the rotational axis of the erecting base with respect to the scanning plane and leading out the treatment tool from the treatment tool channel in a direction obliquely intersecting the scanning plane, for example as in JP2007-215634A and JP2010-4945A.

However, even in this case, the intersection angle between the treatment tool and the scanning plane changes according to the erection angle of the erecting base (the rotation angle around the rotational axis of the erecting base), that is, the lead-out angle of the treatment tool from the treatment tool lead-out port. Therefore, if the tilt angle when the rotational axis of the erecting base is obliquely tilted with respect to the scanning plane is fixed, there is a problem in that the erection angle of the erecting base at which the treatment tool can be favorably guided into the scanning region is limited to an extremely small range. In order to solve this problem, the tilt angle of the rotational axis of the erecting base may be changed according to the erection angle of the erecting base. However, such a change operation by an operator places a large burden to the operator, and a mechanism for changing the tilt angle of a rotational axis is also required, there is a problem in that configuration becomes complicated.

The invention has been made in view of such circumstances, and an object thereof is to provide an ultrasonic endoscope that can reduce a non-used region of a tip part of an insertion section to reduce the diameter of the tip part or the like and can favorably guide a treatment tool into a scanning region of an ultrasonic observation portion over a wide range of the erection angle (the erection angle of an erecting base) of the treatment tool from a treatment tool lead-out port without causing an increase in operational burden or complication of configuration.

In order to achieve the above object, an ultrasonic endoscope of the invention includes an insertion section to be inserted into a body cavity; an ultrasonic observation portion located on a tip side of the insertion section and having an ultrasonic transducer that has a plane parallel to the longitudinal axis of the insertion section as a scanning plane; a treatment tool lead-out port arranged further toward a base end side than the ultrasonic observation portion, provided so as to be capable of leading out a treatment tool inserted through a treatment tool channel of the insertion section, a central axis of the treatment tool lead-out port being arranged at a position apart in a normal direction from the scanning plane; and an erecting base arranged at the treatment tool lead-out port, configured so as to be rotatable around a rotational axis vertical to the scanning plane, changeably adjusting the erection angle of the treatment tool led out of the treatment tool lead-out port, and having a guide surface that bends the treatment tool led out of the treatment tool lead-out port in the direction of the scanning plane.

According to the invention, since the central axis of the treatment tool lead-out port can be shifted and arranged at a position apart in the normal direction from the scanning plane of the ultrasonic observation portion, a non-used region of the tip part can be reduced to decrease the diameter of the tip part or the like. Additionally, since the erecting base is rotated around the rotational axis orthogonal to the scanning plane so as to change the erection angle of the treatment tool, and the treatment tool led out of the treatment tool lead-out port is bent in the direction of the scanning plane at a constant inclination angle by the guide surface irrespective of the erection angle, the intersection angle between the treatment tool and the scanning plane becomes a constant angle irrespective of the erection angle of the treatment tool, and the treatment tool led out of the treatment tool lead-out port is favorably guided into a range near the scanning plane of the ultrasonic observation portion, that is, the range of the scanning region of the ultrasonic observation portion over a wide range of the erection angle of the treatment tool.

In the invention, a form can be adopted in which the ultrasonic observation portion has a region having a predetermined width in a direction orthogonal to the scanning plane as a scanning region of an ultrasonic wave transmitted and received by the ultrasonic transducer, and the guide surface of the erecting base guides a lead-out portion of the treatment tool led out of the treatment tool lead-out port into the scanning region.

According to this, the treatment tool is favorably observed by an ultrasonic image in a wide range of the erection angle due to bending by the guide surface of the erecting base.

In addition, the scanning region observed as an ultrasonic image by the ultrasonic observation portion is a region having a three-dimension spread that is irradiated with an ultrasonic wave. In the present specification, a plane passing through the center of the scanning region is referred to as the scanning plane.

In the invention, a form can be adopted in which an observation optical system is arranged at a side portion of the treatment tool lead-out port further toward the base end side than the ultrasonic observation portion. By arranging the observation optical system in this way, the treatment tool lead-out port including the erecting base that occupies a large region in the tip part and the observation optical system can be arranged in a well-balanced manner at the tip part, and the diameter of the tip part can be reduced.

In the invention, a form can be adopted in which the ultrasonic endoscope further includes two erecting bases arranged at respective treatment tool lead-out ports of two treatment tool channels, and at least one erecting base of the two erecting bases includes the guide surface. Since it is possible to shift and arrange the treatment tool lead-out port from a position on the same plane as the scanning plane, it is possible to arrange the two erecting bases, and it is possible to provide the two treatment tool channels in the ultrasonic endoscope.

In the invention, a form can be adopted in which one erecting base of the two erecting bases is arranged further toward the base end side than the other erecting base. According to this, a situation in which the treatment tools led out via the two erecting bases, respectively, overlap each other completely on an ultrasonic image and one treatment tool is not observed can be prevented.

According to the invention, a non-used region of the tip part of the insertion section can be reduced to decrease the diameter of the tip part or the like and a treatment tool can be favorably guided into the scanning region of the ultrasonic observation portion over a wide range of the erection angle (the erection angle of the erecting base) of the treatment tool from the treatment tool lead-out port without causing an increase in operation burden or complication of configuration.

### BRIEF DESCRIPTION OF THE DRAWINGS

Fig. 1 is an appearance configuration view showing the overall configuration of an ultrasonic inspection system using an ultrasonic endoscope to which the invention is applied.
Fig. 2 is a schematic configuration view showing the overall configuration of the ultrasonic inspection system using the ultrasonic endoscope to which the invention is applied.
Fig. 3 is a perspective view of a tip rigid part of the ultrasonic endoscope.
Fig. 4 is a plan view (top view) of the tip rigid part of the ultrasonic endoscope.
Fig. 5 is a side view of the tip rigid part of the ultrasonic endoscope.
Fig. 6 is a front view of the tip rigid part of the ultrasonic endoscope.
Fig. 7 is an enlarged perspective view showing an erecting base housing portion of a treatment tool lead-out portion.
Fig. 8 is a perspective view showing only the erecting base arranged in the erecting base housing portion of the treatment tool lead-out portion.
Figs. 9A to 9D are four side views showing only the erecting base arranged in the erecting base housing portion of the treatment tool lead-out portion where Fig. 9A is a front view, Fig. 9B is a plan view, Fig. 9C is a right side view, and Fig. 9D is a left side view.
Fig. 10 is a plan view of the tip rigid part showing a scanning region.
Fig. 11 is a side view of the tip rigid part showing the scanning region.
Fig. 12 is a front view of the tip rigid part showing the scanning region.
Fig. 13 is a perspective view showing the relationship between a locus drawn by a lead-out portion of the treatment tool and the scanning region in a case where the erection angle of the erecting base is changed.
Fig. 14 is a plan view showing the relationship between the locus drawn by the lead-out portion of the treatment tool in a case where the erection angle of the erecting base is changed.
Fig. 15 is a plan view showing the relationship between the locus drawn by the lead-out portion of the treatment tool and the scanning region in a case where the erection angle of the erecting base is changed.
Fig. 16 is a bottom view showing the relationship between the locus drawn by the lead-out portion of the treatment tool and the scanning region in a case where the erection angle of the erecting base is changed.
Figs. 17A to 17C are cross-sectional views showing forms of scanning regions at positions of cross-sections including the lead-out portion of the treatment tool at an arbitrary erection angles (an arbitrary lead-out angle of the treatment tool) of the erecting base.
Fig. 18 is a perspective view showing the relationship between a locus drawn by a lead-out portion of a treatment tool and a scanning region in a case where the erection angle of an erecting base is changed in a related-art form.
Fig. 19 is a graph showing the outline of the relationship of between the lead-out angle and intersection angle of the treatment tool in the related art.
Fig. 20 is a graph showing the outline of the relationship of between the lead-out angle and intersection angle of the treatment tool in the invention.
Fig. 21 is a plan view showing the arrangement of constituent elements of the tip rigid part of another form (second embodiment).
Fig. 22 is a plan view showing a modification example of the second embodiment of Fig. 21.
Fig. 23 is a front view schematically showing the tip rigid part.

### DESCRIPTION OF THE PREFERRED EMBODIMENTS

Preferred embodiments of an ultrasonic endoscope related to the invention will be described below in detail with reference to the accompanying drawings.

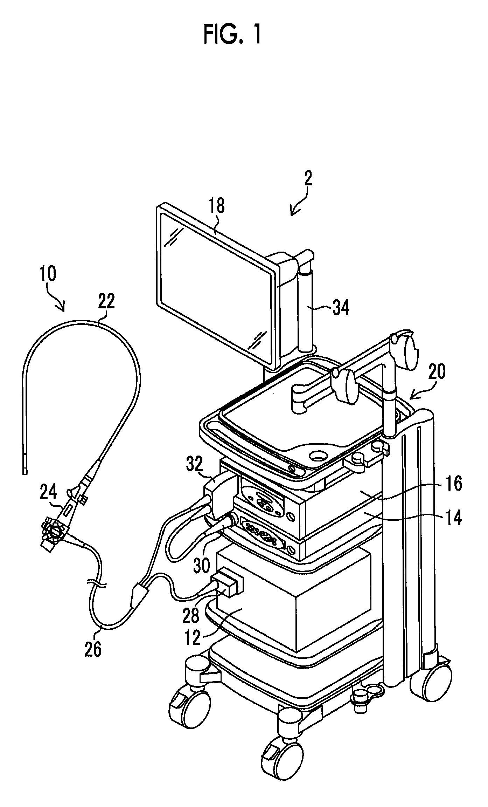

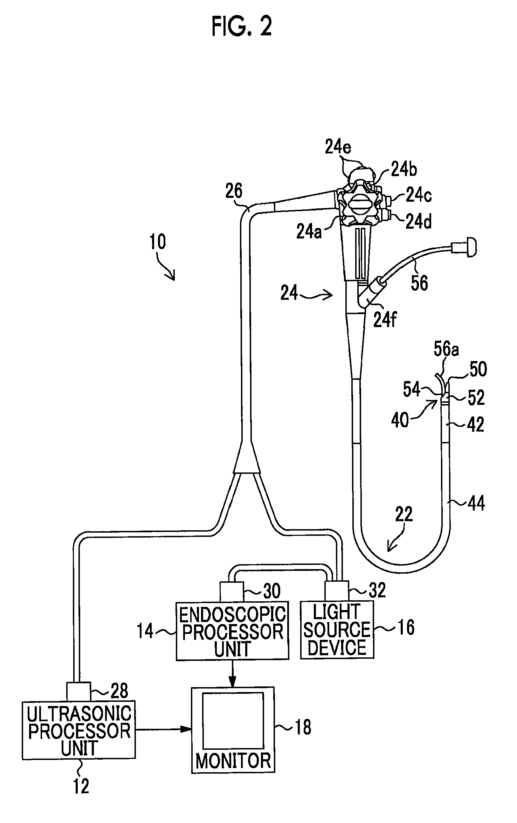

Figs. 1 and 2 are an appearance configuration view and a schematic configuration view showing the overall configuration of an ultrasonic inspection system using an ultrasonic endoscope to which the invention is applied.

As shown in these drawings, an ultrasonic inspection system 2 is configured to include an ultrasonic endoscope 10 that takes the endoscopic image (optical observation image) and an ultrasonic image (tomographic image by an ultrasonic wave) within a body cavity, an ultrasonic processor unit 12 that generates the ultrasonic image, an endoscopic processor unit 14 that generates the endoscopic image, a light source device 16 that supplies illumination light for illuminating the inside of the body cavity to the ultrasonic endoscope 10, and a monitor 18 that displays the endoscopic image or the ultrasonic image.

The ultrasonic endoscope 10 is constituted by an insertion section 22 to be inserted into the body cavity, an operation section 24 coupled to a base end portion of the insertion section 22, and a universal cord 26 that has one end connected to the operation section 24. An ultrasonic connector 28 connected to the ultrasonic processor unit 12, an endoscopic connector 30 connected to the endoscopic processor unit 14, and a light source connector 32 connected to the light source device 16 are provided at the other end portion of the universal cord 26. The ultrasonic endoscope 10 is detachably connected to the ultrasonic processor unit 12, the endoscopic processor unit 14, and the light source device 16 via the connectors 28, 30, and 32, respectively.

The ultrasonic processor unit 12, the endoscopic processor unit 14, and the light source device 16 are placed on a cart 20 with casters as shown in Fig. 1, and can be integrally and freely moved. The cart 20 is provided with a supporting post 34, and the monitor 18 is held by the supporting post 34. The supporting post 34 includes a rotating mechanism and a height-adjusting mechanism, and can adjust the direction and the height of a screen of a monitor 18.

The ultrasonic endoscope of the present embodiment is a convex ultrasonic endoscope, and is constituted by the insertion section 22, the operation section 24, and the universal cord 26 as mentioned above.

As shown in Fig. 2, the insertion section 22 to be inserted into the body cavity is constituted by a tip rigid part (tip part) 40 formed of a tip member, a bendable bending part 42 that is provided continuously with a base end side of the tip rigid part 40, and a soft part 44 that couples a base end side of the bending part 42 and a tip side of the operation section 24 together, and is small-diametered, lengthy, and has flexibility, sequentially from a tip side thereof. Additionally, the inside of the insertion section 22 is formed with a treatment tool channel (not shown) that allows insertion from the base end to the tip.

The tip rigid part 40 is provided with an ultrasonic observation portion 50, an endoscopic observation portion 52, and a treatment tool lead-out portion (treatment tool lead-out port) 54 as will be described below in detail.

The ultrasonic observation portion 50 has an ultrasonic transducer in which an ultrasonic wave transmitting and receiving surface is formed by arraying a plurality of ultrasonic transducers that transmit and receive an ultrasonic wave. An ultrasonic signal for generating a tomographic image of a cellular tissue that is present further in the depth direction than the wall of the body cavity as an ultrasonic image is acquired by the ultrasonic observation portion 50.

The endoscopic observation portion 52 is constituted by a constituent member, an imaging element, its peripheral circuit, or the like of an observation optical system and an illumination optical system, and an imaging signal for optically imaging the wall surface of the body cavity to display an endoscopic image for observation is acquired by the ultrasonic observation portion 50.

The treatment tool lead-out portion (treatment tool lead-out port) 54 includes an erecting base that leads out tip parts of various treatment tools 56, which are inserted through the treatment tool channel, into the body cavity, and is capable of changing the lead-out direction of the treatment tools 56 from the treatment tool channel as will be described below in detail.

The operation section 24 coupled to a base end side of the insertion section 22 is a constituent portion gripped by an operator, and includes an operating member for performing various operations. For example, the operation section 24 includes an angle knob 24a that operates to bend the bending part 42 of the insertion section 22 vertically and horizontally, an erecting lever 24b that erects the erecting base, a suction button 24c for performing a suction operation, an air/water supply button 24d of for performing an air/water supply operation, a plurality of operating members 24e that perform display switching of the monitor, a freeze instruction or release instruction of a display image, or the like.

Additionally, a treatment tool insertion port 24f for inserting the various treatment tools 56 into the treatment tool channel is provided on the tip side of the operation section 24 so as to protrude therefrom.

The universal cord 26 that has one end connected to the operation section 24 has therein various signal lines that transmit an electrical signal or the like, a light guide that transmits illumination light, or the like. A tip portion of the universal cord 26 is provided with the ultrasonic connector 28 connected to the ultrasonic processor unit 12, the endoscopic connector 30 connected to the endoscopic processor unit 14, and the light source connector 32 connected to the light source device 16 as described above.

Thereby, the ultrasonic observation portion 50 of the tip rigid part 40 of the insertion section 22 and the ultrasonic processor unit 12 are connected by a signal line inserted through the insertion section 22, the operation section 24, and the universal cord 26, and the endoscopic observation portion 52 of the tip rigid part 40 of the insertion section 22 and the endoscopic processor unit 14 are connected by a signal line inserted through the insertion section 22, the operation section 24, and the universal cord 26. Additionally, the illumination optical system of the tip rigid part 40 of the insertion section 22 and the light source device 16 are connected by the light guide inserted through the insertion section 22, the operation section 24, and the universal cord 26.

The ultrasonic processor unit 12 drives an ultrasonic transducer (to be described below) of the ultrasonic observation portion 50 to thereby acquire from the ultrasonic observation portion 50 an electrical signal (ultrasonic signal) obtained by transmitting a predetermined frequency of an ultrasonic wave toward an observation object and receiving an ultrasonic wave reflected from the observation object, and performs various kinds of signal processing to thereby generate a video signal for an ultrasonic image.

The endoscopic processor unit 14 controls driving of the imaging element of the endoscopic observation portion 52 of the ultrasonic endoscope 10 to thereby acquire an imaging signal transmitted from the imaging element, and performs various kinds of signal processing to generate a video signal for an endoscopic image.

The light source device 16 supplies illumination light emitted from the illumination optical system of the tip rigid part 40 in order to illuminate an observation visual field range defined by the endoscopic observation portion 52.

The monitor 18 receives the respective video signals generated by the ultrasonic processor unit 12 and the endoscopic processor unit 14 to display the ultrasonic image or the endoscopic image. As for the display of the ultrasonic image or endoscopic image, it is possible to appropriately only switch any one image of the images to display the image on the monitor 18, simultaneously display both the images, or the like. In addition, a monitor for displaying the ultrasonic image and a monitor for displaying the endoscopic image may be separately provided, or these ultrasonic images and endoscopic image may be displayed in other arbitrary forms.

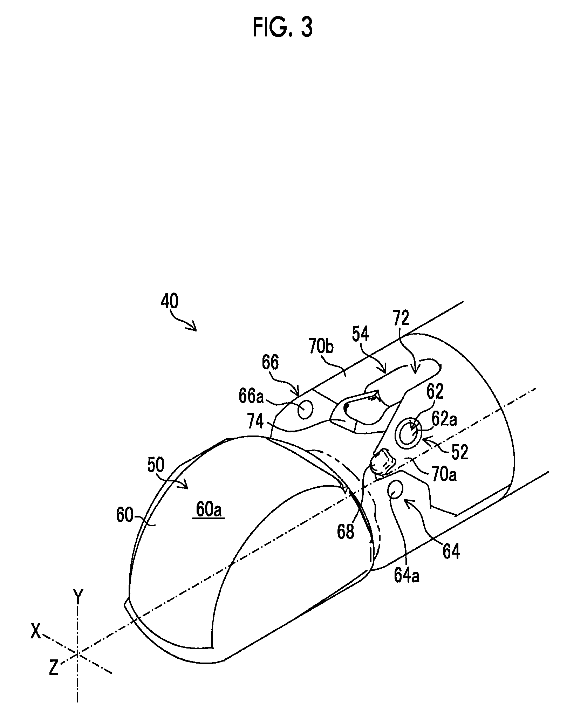

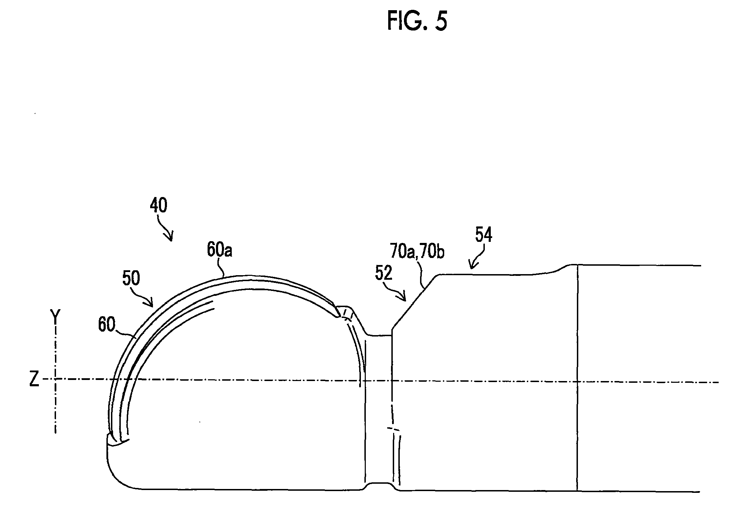

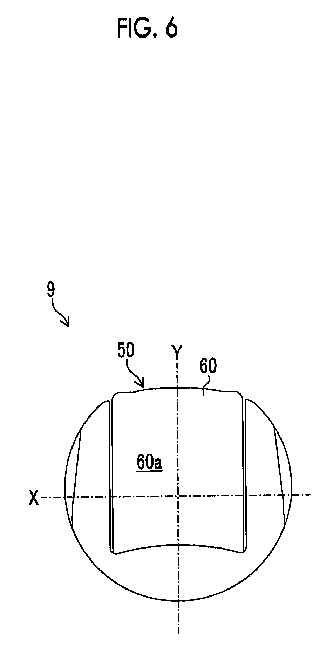

Next, the configuration of the tip rigid part 40 of the ultrasonic endoscope 10 will be described in detail. Figs. 3, 4, 5, and 6 are a perspective view, a plan view, a side view, and a front view of the tip rigid part 40, respectively.

As shown in these drawings, the tip rigid part 40 is provided with the ultrasonic observation portion 50, the endoscopic observation portion 52, and the treatment tool lead-out portion (treatment tool lead-out port) 54.

The ultrasonic observation portion 50 has an ultrasonic transducer 60 that is configured by arraying ultrasonic transducers along the direction of an insertion axis (central axis) Z of the insertion section 22 that serves as the longitudinal axis of the tip rigid part 40. The respective ultrasonic transducers that constitutes the ultrasonic transducer 60 is arrayed from a position near the tip of the tip rigid part 40 toward the base end side (back side) in the direction of the insertion axis Z, and an ultrasonic wave transmitting and receiving surface 60a that transmits and receives an ultrasonic wave has a convexly curved shape toward the direction of the insertion axis Z. The respective ultrasonic transducers of the ultrasonic transducer 60 are sequentially driven so as to perform ultrasonic electronic scanning.

In addition, if a horizontal axis in a right-and-left direction orthogonal to the insertion axis Z is defined as X and a vertical axis in an up-and-down direction is defined as Y, the ultrasonic wave transmitting and receiving surface 60a of the ultrasonic transducer 60 is arranged so as to be substantially bilaterally symmetrical with respect to a plane (YZ plane) including the insertion axis Z.

The endoscopic observation portion 52 is constituted by a constituent member, an imaging element, or the like of the observation optical system 62 or the illumination optical systems 64 and 66, and is arranged at a position that is closer to the base end side than the ultrasonic observation portion 50 and is away from the treatment tool lead-out portion 54.

Tip portions of both left and right side portions of the treatment tool lead-out portion 54 are provided with inclined surface portions 70a and 70b that incline at predetermined angles with respect to a plane orthogonal to the insertion axis Z, and an observation window 62a of the observation optical system 62 and an illumination window 64a of one illumination optical system 64 of the two illumination optical systems 64 and 66 are disposed in the inclined surface portion 70a on the left from the base end side toward the tip side. An illumination window 66a of the other illumination optical system 66 is disposed in the inclined surface portion 70b on the right from the base end side toward the tip side.

The observation optical system 62 includes an optical system member (not shown) that takes in light from a subject in the observation visual field range from the observation window 62a, and focuses a subject image inside the tip rigid part 40. An imaging element (not shown) that captures the subject image focused by the observation optical system 62 to generate an imaging signal is arranged inside the tip rigid part 40.

The illumination optical systems 64 and 66 include optical system members that diffuse and emit illumination light transmitted through the light guide from the light source device 16 to the observation visual field range via the illumination windows 64a and 66a, respectively.

In addition, a cleaning nozzle 68 that ejects a liquid or a gas toward the observation window 62a is provided near the observation window 62a on the inclined surface portion 70a.

The treatment tool lead-out portion (treatment tool lead-out port) 54 is provided further toward the base end side than the ultrasonic transducer 60 of the ultrasonic observation portion, and includes an erecting base housing portion 72 that is a concave space that is formed so as to communicate with a conduit opening of the treatment tool channel (not shown). An erecting base 74 capable of changing the lead-out direction of a treatment tool led out of the conduit opening of the treatment tool channel from the treatment tool lead-out portion 54 is rotatably fixed to the erecting base housing portion 72.

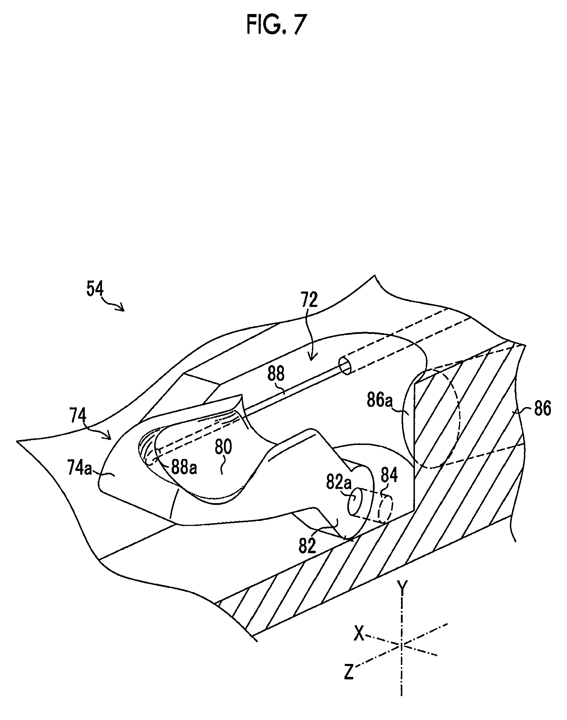

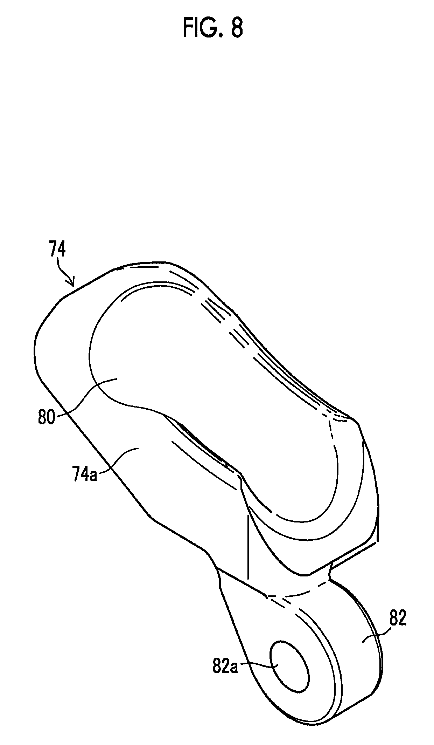

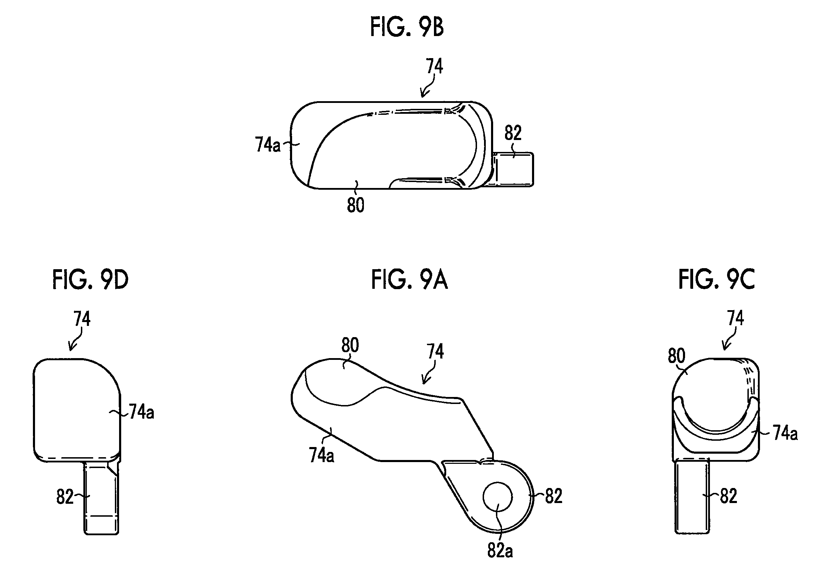

Fig. 7 is an enlarged perspective view showing the erecting base housing portion 72 of the treatment tool lead-out portion 54, and Fig. 8 and Figs. 9A to 9D are a perspective view and four side views showing only the erecting base 74 in an enlarged manner. As shown in these drawings, the erecting base 74 has a guide surface 80 (to be described below in detail), which guides a treatment tool in a predetermined lead-out direction, on the upper side of a main body 74a, and a shaft hole portion 82 having a shaft hole 82a, on the lower side of main body 74a.

On the other hand, as shown in Fig. 7, a shaft member 84 is provided on an inner wall surface (side wall surface) (not shown) on the left from the base end side toward the tip side among inner wall surfaces of the erecting base housing portion 72 so as to protrude toward the direction of the horizontal axis X, and the shaft member 84 is inserted through the shaft hole 82a of the erecting base 74. Thereby, the erecting base 74 is rotatably supported by the shaft member 84, with the shaft member 84 in the direction of the horizontal axis X as a rotational axis.

Additionally, an opening (conduit opening) 86a of the conduit 86 of a treatment tool channel is provided in an inner wall surface on the base end side among the inner wall surfaces of the erecting base housing portion 72, and the erecting base 74 and the conduit 86 (conduit opening 86a) of the treatment tool channel are arranged so that the center position of the width of the erecting base 74 in the right-and-left direction and the center position of the conduit opening 86a of the treatment tool channel substantially coincide with each other in the direction of the horizontal axis X.

A tip portion 88a of an operating wire 88 is attached to a side surface of the erecting base 74 on the right from the base end side toward the tip side. The operating wire 88 is operated so as to be pushed and pulled by the operation of the erecting lever 24b (refer to Fig. 2) of the operation section 24, and the erecting base 74 is rotated with the shaft member 84 as a rotational axis by the push-pull operation of the operating wire 88 so that the erection angle (rotation angle around the rotational axis) of the erecting base 74 is changed.

According to this, a treatment tool led out of the conduit opening 86a of the treatment tool channel is guided in the predetermined lead-out direction along the guide surface 80 of the erecting base 74, and is led out of the opening (treatment tool lead-out portion 54) of the erecting base housing portion 72. Accordingly, the lead-out direction of the treatment tool led out of the treatment tool lead-out portion 54 can be changed by changing the erection angle of the erecting base 74 by the erecting lever 24b.

Subsequently, the positional relationship between the erecting base 74 of the treatment tool lead-out portion (treatment tool lead-out port) 54 and the ultrasonic transducer 60 of the ultrasonic observation portion 50, and the guide surface 80 of the erecting base 74 will be described in detail.

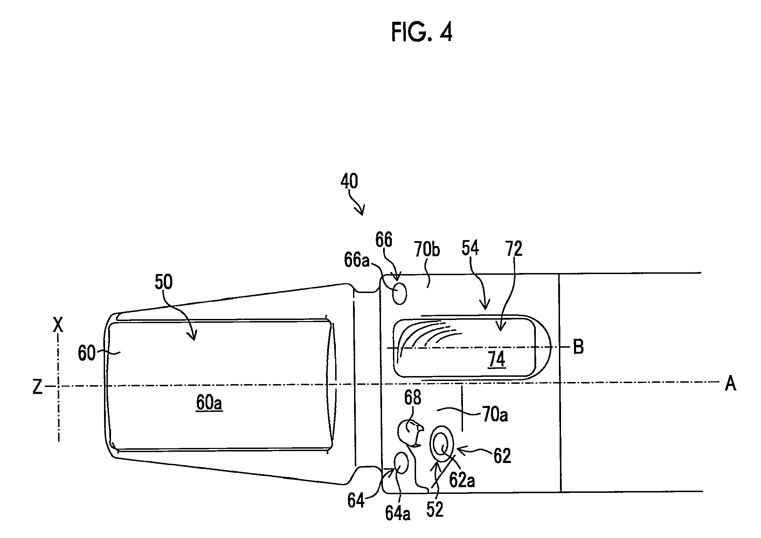

On the plan view of the tip rigid part 40 of Fig. 4, the position of a central axis A passing through the center of the width of the ultrasonic transducer 60 in the right-and-left direction (the direction of the horizontal axis X) substantially coincides with the position of the insertion axis Z, and the ultrasonic transducer 60 is arranged at a position where the ultrasonic transducer has a shape that is substantially symmetrical with respect the insertion axis Z.

Here, in the present embodiment, a scanning plane that is the center plane of a scanning region by the ultrasonic transducer 60, to be described in detail, is formed on a vertical plane (YZ plane) including the central axis A of the ultrasonic transducer 60. Accordingly, the scanning plane is formed on a vertical plane including the insertion axis Z.

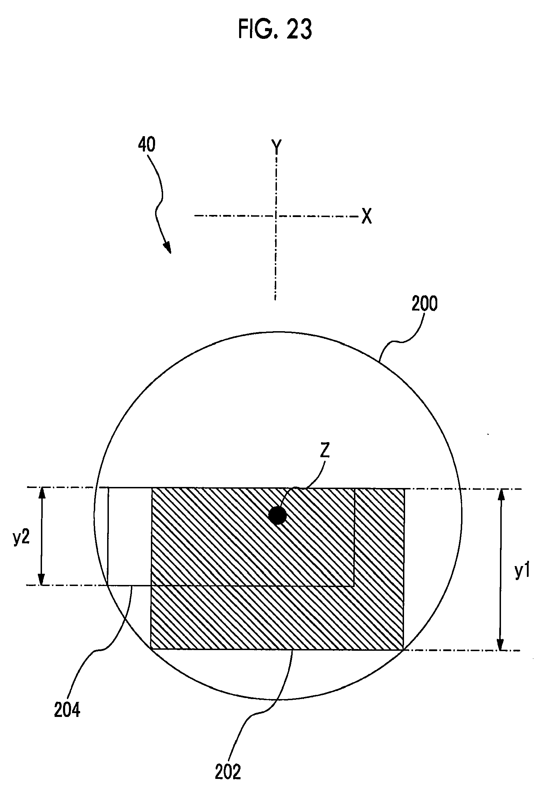

By arranging the central axis A of the ultrasonic transducer 60 so as to substantially coincide with the position of the insertion axis Z with respect to the right-and-left direction (the direction of the horizontal axis X), the ultrasonic transducer 60 that is as large as possible can be used. That is, if the tip rigid part 40 is schematically shown from the front side, as shown in a front view of Fig. 23, a region of a base-end-side portion of the tip rigid part 40 where the endoscopic observation portion 52 and the treatment tool lead-out portion 54 are arranged is expressed by a circular frame 200 centered on the insertion axis Z.

On the other hand, in a case where the region of the base-end-side portion of the tip rigid part 40 where the ultrasonic observation portion 50 is arranged is shown by a rectangular (oblong) frame and the ultrasonic observation portion 50 is settled within the region of the base-end-side portion of the tip rigid part 40, the size of the rectangular frame of which base-end-side angled portions touch the circular frame 200 becomes the maximum as for the size of the ultrasonic observation portion 50. At this time, if the central axis A of the ultrasonic transducer 60 is arranged so as to substantially coincide with the position of the insertion axis Z with respect to the right-and-left direction as in the present embodiment, the size of a rectangular frame 202 of this drawing becomes the maximum as for the size of the ultrasonic observation portion 50, and if the central axis A of the ultrasonic transducer 60 is arranged so as to shift from the position of the insertion axis Z, the size of a rectangular frame 204 of this drawing becomes the maximum as for the size of the ultrasonic observation portion 50.

If the rectangular frames 202 and 204 are compared with each other, as can also be seen from the fact that the length y1 of the rectangular frame 202 in the up-and-down direction (the direction of the vertical axis Y) is greater than the length y2 of the rectangular frame 204 in the direction of vertical axis Y, a space where the ultrasonic observation portion 50 can be arranged becomes greater as the central axis A of the ultrasonic transducer 60 is arranged so as to be as close to the position of the insertion axis Z as possible, and a larger ultrasonic transducer can be arranged correspondingly.

Thus, in the present embodiment, by arranging the ultrasonic transducer 60 so that the central axis A of the ultrasonic transducer 60 substantially coincides with the position of the insertion axis Z with respect to the right-and-left direction, the ultrasonic transducer 60 that is as large as possible can be used. However, positions in the horizontal positions between the central axis A of the ultrasonic transducer 60, and the insertion axis Z are not necessarily made to coincide with each other.

On the other hand, as shown in Fig. 4, the position of a central axis B passing through the center of the width of the treatment tool lead-out portion 54 (in the present embodiment, also coincides with a central axis passing through the center of the width of the erecting base 74 in the right-and-left direction) in the right-and-left direction does not coincide with the position of the insertion axis Z, and is arranged at a position apart from the insertion axis Z by a predetermined distance so as to be substantially parallel to the insertion axis Z. That is, the central axis B of the treatment tool lead-out portion 54 is arranged at a position apart in a normal direction from the scanning plane.

In this way, by arranging the position of the central axis B of the treatment tool lead-out portion 54 (erecting base 74) so as to shift from the position of the central axis A of the ultrasonic transducer 60, that is, a position on the scanning plane to be described in detail, to thereby arrange the erecting base 74 and the ultrasonic transducer 60 at different positions with respect to the direction of the horizontal axis X, a wasteful non-used region on the side of one side portion where the observation optical system 62 or the imaging element is not arranged, out of both side portions of the treatment tool lead-out portion 54, is reduced, and the diameter of the tip rigid part 40 is reduced.

In addition, usually, the arrangement of the insertion axis Z or respective constituent portions such as the treatment lead-out portion 54 is described with the position of the scanning plane as a reference. However, in the present embodiment, the central axis A of the ultrasonic transducer 60 and the position of the insertion axis Z in the right-and-left direction coincides with each other, and the scanning plane is formed on the vertical plane (YZ plane) including the central axis A and the insertion axis Z. Therefore, the arrangement of the respective constituent portions such as the treatment tool lead-out portion 54 will mainly be described with the insertion axis Z as a reference. In the present invention, the central axis A and the position of the insertion axis Z in the right-and-left direction may not necessarily coincide with each other as described above, and a form in which the scanning plane is not formed on the vertical plane including the central axis A is also included.

Accordingly, the positional relationship between the insertion axis Z and the respective constituent portions that is satisfied in the present embodiment merely shows one form of a case where the central axis A and the position of the insertion axis Z in the right-and-left direction coincide with each other and the scanning plane is formed on the vertical plane (YZ plane) including the insertion axis Z and the insertion axis A, and the positional relationship between the insertion axis Z and the respective constituent portions is not limited to that of the present embodiment.

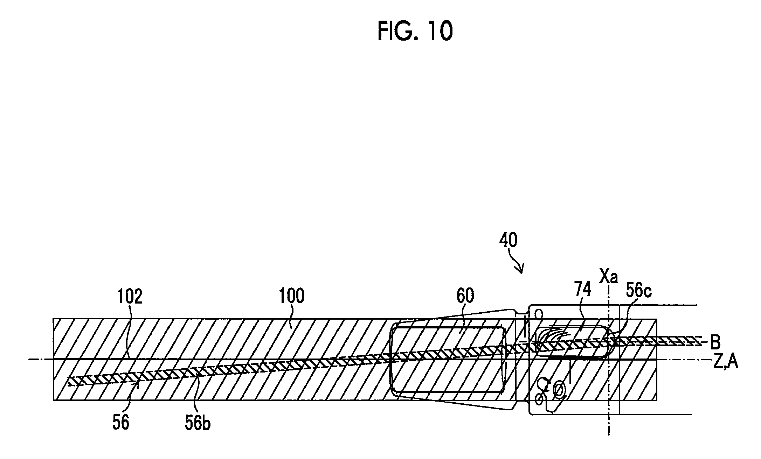

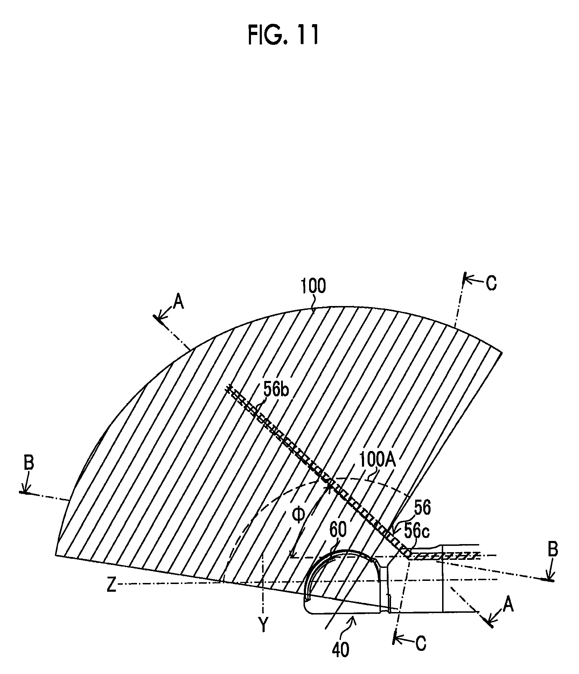

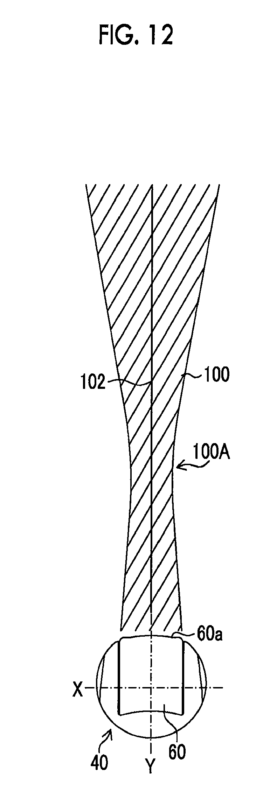

A scanning region observed as an ultrasonic image by the ultrasonic transducer 60, that is, a scanning region 100 that is irradiated with ultrasonic waves sequentially transmitted and received by the electronic scanning of the ultrasonic transducer 60, as shown by hatched regions in a plan view, a side view, and a front view of the tip rigid part 40 of Figs. 10, 11, and 12 shows a sectoral range (refer to Fig. 11) when the ultrasonic transducer 60 is seen from a side surface side and becomes regions (refer to Figs. 10 and 12) having predetermined spreads in the right-and-left direction (the direction of the horizontal axis X) as seen from a front side and a top side. If a plane passing through the center position of the scanning region 100 in the right-and-left direction is referred to as a scanning plane 102, in the present embodiment, the scanning plane 102 is formed at a position that substantially coincides with the vertical plane (YZ plane) including the central axis A of the ultrasonic transducer 60, and the scanning region 100 is formed as a region having a predetermined spread in the right-and-left direction (the direction of the horizontal axis X) with respect to the scanning plane 102. In addition, Fig. 12 shows a scanning region on a cross-section orthogonal to the insertion axis Z.

Additionally, since the ultrasonic transducer 60 emits an ultrasonic beam so that a point with a predetermined distance from the ultrasonic wave transmitting and receiving surface 60a becomes a focus, the width of the scanning region 100 in the direction of the horizontal axis X, as shown in Fig. 12, becomes gradually smaller as the distance from the ultrasonic wave transmitting and receiving surface 60a of the ultrasonic transducer 60 becomes longer, and becomes gradually larger after being converged so as to become a minimum width at a distance that becomes a focus. The position of the convergence region where the scanning region 100 has the minimum width is shown by reference numeral 100A in Figs. 11 and 12.

Here, if the erecting base 74 guides a treatment tool from the treatment tool channel such that the treatment tool is parallel to the scanning plane 102 and leads out of the treatment tool lead-out portion 54, the treatment tool deviates from the scanning region 100 at least near the convergence region 100A of the scanning region 100. As the shift length in the direction of the horizontal axis X between the central axis B of the treatment tool lead-out portion 54 (erecting base 74) and the central axis A of the ultrasonic transducer 60 is large, a range where a treatment tool led out of the treatment tool lead-out portion 54 does not pass through the scanning region 100 becomes large.

Thus, the guide surface 80 of the erecting base 74 shown in Fig. 7 or the like is formed so as to guide a treatment tool led out of the conduit opening 86a of the treatment tool channel not only in an in-plane direction parallel to the scanning plane 102 but also a direction inclined to the scanning plane 102 side, that is, a direction intersecting the scanning plane 102 and to lead out the treatment tool in a direction passing through the range of the scanning region 100 in the convergence region 100A of the scanning region 100. Thereby, a treatment tool led out of the treatment tool lead-out port 54 favorably passes through the range the scanning region 100 irrespective of the erection angle (hereinafter referred to as lead-out angle) of the treatment tool from the treatment tool lead-out portion 54 according to the erection angle of the erecting base 74.

As shown in Figs. 7 to 9D, the guide surface 80 of the erecting base 74 is formed as an inner wall surface of a groove having a substantially circular-arc cross-section, and is formed by curving the groove in a direction in which the groove gradually approaches the scanning plane 102 side from the base end side of the erecting base 74 toward the tip side. However, the guide surface 80 is not limited to this, and may be formed by a curved surface, a planar surface, or the like that is made to incline gradually toward the scanning plane 102 side as it shifts from the base end side of the erecting base 74 to the tip side. A treatment tool from the treatment tool channel is guided in a direction intersecting the scanning plane 102 by such a guide surface 80.

In addition, the structure (the shape or the like of the rotating mechanism or the guide surface 80) of the erecting base 74 is not limited to that of the present embodiment, and the erecting base 74 may be rotated around on the rotational axis (the direction of the horizontal axis X) in a direction orthogonal to the scanning plane 102 so as to change the erection angle, and may be configured so as to guide a treatment tool from the treatment tool channel in the direction intersecting the scanning plane 102 according to the shape or structure of the guide surface.

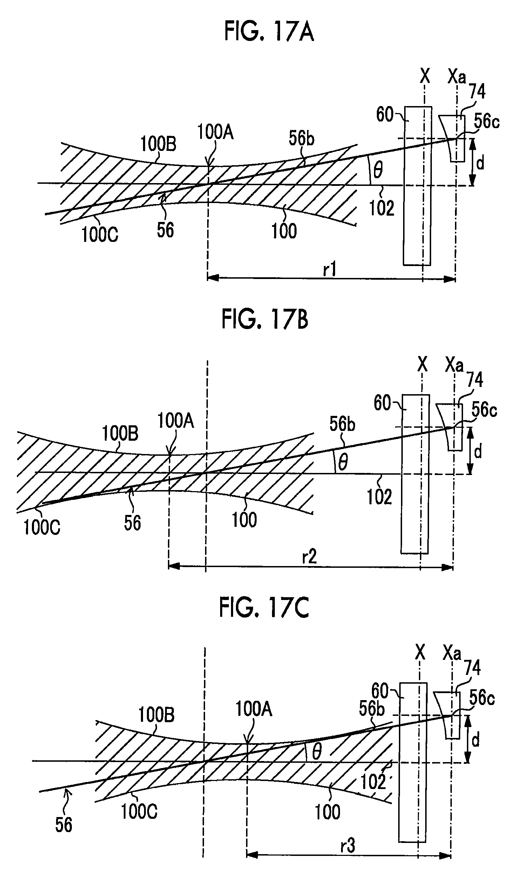

If description is made about the lead-out direction of a treatment tool, the lead-out angle (erection angle) of the treatment tool that changes according to the erection angle of the erecting base 74, as shown in a treatment tool 56 shown in Fig. 11, shows an angle φ between a lead-out portion 56b led out of the treatment tool lead-out portion 54 (conduit opening 86a of the treatment tool channel) with a bending position 56c bent by the erecting base 74 as an origin and a horizontal plane (XZ plane), and an angle between the lead-out portion 56b and a vertical plane (YZ plane and the scanning plane 102) is referred to as an intersection angle θ (shown in Figs. 17A to 17C to be described below). If the erection angle of the erecting base 74 changes as described later, the lead-out angle φ of the treatment tool 56 changes, but the intersection angle θ does not change.

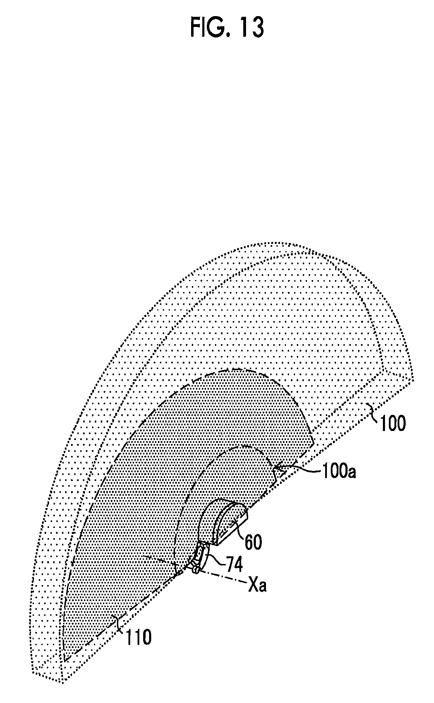

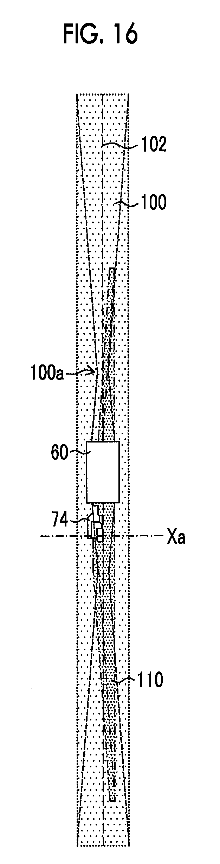

Figs. 13 to 16 are views showing a relationship between a locus 110 drawn by the lead-out portion 56b of the treatment tool 56 led out of the treatment tool lead-out portion 54 in a case where the erection angle of the erecting base 74 is changed to change the lead-out angle φ of the treatment tool 56 shown in Figs. 10 and 11, and the scanning region 100 (and the scanning plane 102).

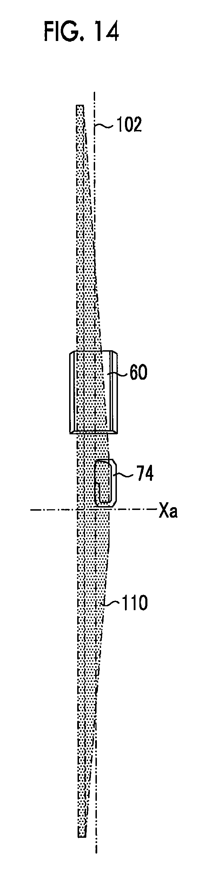

In addition, only the ultrasonic transducer 60 and the erecting base 74 among the constituent elements of the tip rigid part 40 are shown in Figs. 13 to 16. Figs. 13, 15, and 16 are respectively a perspective view, a plan view (top view), and a bottom view showing the relationship between the locus 110 drawn by the lead-out portion 56b of the treatment tool 56, and the scanning region 100, and Fig. 14 is a plan view showing only the locus 110 drawn by the lead-out portion 56b of the treatment tool 56.

The lead-out portion 56b of the treatment tool 56 is rotated around a rotational axis Xa in the direction of the horizontal axis X passing through the bending position 56c (refer to Figs. 10 and 11) bent by the erecting base 74. In addition, the rotational axis Xa of the treatment tool 56 substantially coincides with the position and direction of the rotational axis (shaft member 84) of the erecting base 74, and these rotational axes will be described as the rotational axis Xa without being distinguished from each other.

The lead-out portion 56b of the treatment tool 56 is lead out in the direction intersecting at the intersection angle θ with respect to the scanning plane 102 by the guide surface 80 of the erecting base 74 as described above. As the erecting base 74 is rotated around the rotational axis Xa in the direction of the horizontal axis X, the lead-out portion 56b of the treatment tool 56 is also rotated around the rotational axis Xa, and draws the locus 110 along a conical surface as shown in Figs. 13 to 16. Accordingly, the intersection angle θ between the treatment tool 56 and the scanning plane 102 is maintained at a constant angle with respect to an arbitrary erection angle of the erecting base 74, that is, an arbitrary lead-out angle φ of the treatment tool 56.

The intersection angle θ between the treatment tool 56 and the scanning plane 102 is set to the following angle. Figs. 17A to 17C show cross-sections obtained by cutting the scanning region 100 in a plane that includes the lead-out portion 56b of the treatment tool 56 and is orthogonal to the vertical plane (YZ plane), in a predetermined erection angle of the erecting base 74, that is, in a predetermined lead-out angle φ of the treatment tool 56. In a case where the lead-out angle φ of the treatment tool 56 shown in Fig. 11 is defined as φ1, Fig. 17A is a cross-sectional view seen from the direction of arrow A-A when the scanning region 100 is cut by a plane that has the direction of the lead-out angle φ1 and is vertical to the sheet surface of Fig. 11, Fig. 17B is a cross-sectional view seen from the direction of arrow B-B when the scanning region 100 is cut by a plane that has the direction of a lead-out angle φ2 (φ2<φ1) smaller than the lead-out angle φ1 and is vertical to the sheet surface of Fig. 11, and Fig. 17C is a cross-sectional view seen from the direction of arrow C-C when the scanning region 100 is cut by a plane that has the direction of a lead-out angle φ3 (φ3>φ1>φ2) larger than the lead-out angle φ1 and is vertical to the sheet surface of Fig. 11.

As shown in Figs. 17A to 17C, the forms of the scanning regions 100 on the cross-sections show substantially the same form irrespective of the lead-out angle φ of the treatment tool 56. That is, the width of the scanning region 100 (boundary lines 100B and 100C) in the direction of the horizontal axis X decreases gradually as the scanning region is away from the erecting base 74 (the bending position 56c of the treatment tool 56), and increases gradually if the scanning region exceeds the convergence region 100A.

With respect to the scanning region 100 of such a form, if the intersection angle θ from the scanning plane 102 is set so that the lead-out portion 56b of the treatment tool 56 passes through the range of the scanning region 100 in the convergence region 100A, the lead-out portion 56b of the treatment tool 56 favorably passes through the scanning region 100 over a wide range. Such conditions are satisfied when the intersection angle θ is within a predetermined angle range (an angle range of which the lower limit is θmin and the upper limit is θmax: hereinafter referred to as allowable angle range).

On the other hand, the distance r/cosθ (the distance r from the rotational axis Xa to the convergence region 100A) from the bending position 56c of the treatment tool 56 to the convergence region 100A (position having a minimum width) of the scanning region 100 varies depending on the lead-out angle φ of the treatment tool 56 according to the erection angle of the erecting base 74. For example, the distance r becomes larger as the lead-out angle φ of the treatment tool 56 becomes smaller, and the distance r becomes smaller as the lead-out angle φ of the treatment tool 56 becomes larger. As shown in Figs. 17A to 17C, if respective distances r when the lead-out angles φ of the treatment tool 56 are φ1, φ2, and φ3 (φ2 < φ1 <φ3) is r1, r2, and r3, r3 < r1 < r2 is established.

Therefore, restriction of the upper limit θmax of the allowable intersection angle range becomes more severe (the upper limit θmax becomes smaller) as the lead-out angle φ of the treatment tool 56 becomes smaller, and restriction of the lower limit θmin of the allowable intersection angle range becomes more severe (the lower limit θmin becomes larger) as the lead-out angle φ of the treatment tool 56 becomes larger.

Accordingly, in a case where the range of the lead-out angle of the treatment tool 56 that enables the treatment tool 56 led out of the treatment tool lead-out portion 54 to be effectively observed by an ultrasonic image is an angle range from the lead-out angle φmin (minimum lead-out angle) to the lead-out angle φmax (maximum lead-out angle), in order for the lead-out portion 56b of the treatment tool 56 to pass through the scanning region 100 without deviating from the scanning region 100 in an arbitrary lead-out angle φ within the above angle range, the intersection angle θ may be set so as to be an angle that satisfies the condition of θmin ≤ θ ≤ θmax with respect to the upper limit θmax of the allowable intersection angle range at the minimum lead-out angle φmin, and the lower limit θmin of the allowable intersection angle range at the maximum lead-out angle φmax.

In the present embodiment, since the intersection angle θ is set so as to satisfy the above condition, for example, using the minimum lead-out angle φmin and maximum lead-out angle φmax as the minimum angle and maximum angle of the lead-out angle φ capable of being changed by the adjustment of the erection angle of the erecting base 74, the lead-out portion 56a of the treatment tool 56 passes through the scanning region 100 without deviating from the scanning region 100 irrespective of the lead-out angle φ of the treatment tool 56 from the treatment tool lead-out portion 54.

In order for the intersection angle θ to satisfy the above condition, the characteristics (the range of the scanning region 100) of the ultrasonic transducer 60 may be determined correspondingly, for example, at the time of design and then the intersection angle θ may be determined, or, the intersection angle θ may be determined and then the characteristics of the ultrasonic transducer 60 may be determined correspondingly. The invention is not limited to a specific method.

In addition, in a case where the intersection angle θ is set so as to satisfy the above condition, using the minimum angle and maximum angle of the lead-out angle φ in an angle range limited to a partial range out of an angle range from the minimum angle to the maximum angle in the lead-out angle φ capable of being changed by the adjustment of the erection angle of the erecting base 74 as the minimum lead-out angle φmin and the maximum lead-out angle φmax, it does not mean that the treatment tool 56 never pass through the range of the scanning region 100 at all even in a case where the lead-out angle φ deviates from the angle range from the minimum lead-out angle φmin to the maximum lead-out angle φmax. Additionally, since changes in the allowable intersection angle range that fluctuates according to the lead-out angle φ are not large, the treatment tool 56 passes through the scanning region 100 over a wide range in an arbitrary lead-out angle φ (the wide range angle range of the lead-out angle φ) capable of being changed by the adjustment of the erection angle of the erecting base 74. As a result, ultrasonic observation of the treatment tool 56 led out of the treatment tool lead-out portion 54 can be favorably observed.

Additionally, in a case where the lead-out portion 56b of the treatment tool 56 is configured so as to pass through the scanning region 100 without deviating from the scanning region 100 in an arbitrary lead-out angle φ between the predetermined minimum lead-out angle φmin and the maximum lead-out angle φmax as described above, the shift length d of the central axis B of the erecting base 74 from the scanning plane 102 (refer to Fig. 4) becomes the maximum if the erecting base 74 (the bending position 56c of the treatment tool 56) is arranged at a position that has such an intersection angle φ that the lead-out portion 56b of the treatment tool 56 touches both a boundary line 100C (a boundary line on a side where the erecting base 74 (the bending position 56c of the treatment tool 56) is not present with respect to the scanning plane 102 (face)) of the scanning region 100 at the minimum lead-out angle φmin, and a boundary line 100B (a boundary line (surface) on a side where the erecting base 74 (the bending position 56c of the treatment tool 56) is present with respect to the scanning plane 102) of the scanning region 100 at the maximum lead-out angle φmax. Accordingly, the erecting base 74 can be favorably shifted at least to the above position. Figs. 17A to 17C illustrate a form in which the shift length d becomes the maximum such that the lead-out angle φ2 of Fig. 17B is used as the minimum lead-out angle φmin, the lead-out angle φ3 of Fig. 17C is used as the maximum lead-out angle φmax, the lead-out portion 56b of the treatment tool 56 at the lead-out angle φ2 of Fig. 17B touch the boundary line 100C of the scanning region 100, and the lead-out portion 56b of the treatment tool 56 at the lead-out angle φ3 of Fig. 17C touches the boundary line 100B of the scanning region 100.

Moreover, in the above description, the distance r/cosθ from the bending position 56c of the treatment tool 56 to the convergence region 100A of the scanning region 100 becomes larger as the lead-out angle θ of the treatment tool 56 is smaller, and the intersection angle θ satisfies the condition of θmin ≤ θ ≤ θmax with respect to the upper limit θmax of the allowable intersection angle range at the minimum lead-out angle θmin and the lower limit θmin of the allowable intersection angle range at the maximum lead-out angle θmax.

On the other hand, when taking into consideration a case that is different from the form which the distance r/cosθ from the bending position 56c of the treatment tool 56 to the convergence region 100A of the scanning region 100 becomes larger as the lead-out angle φ of the treatment tool 56 is smaller, the intersection angle θ may satisfy the condition of θmin ≤ θ ≤ θmax, using the upper limit of the allowable intersection angle range when the distance from the bending position 56c of the treatment tool 56 to the convergence region 100A of the scanning region 100 becomes the furthest as the above upper limit θx and using the lower limit of the allowable intersection angle range when the distance from the bending position 56c of the treatment tool 56 to the convergence region 100A of the scanning region 100 becomes the nearest as the above lower limit θx, in the angle range of the lead-out angle φ from the minimum lead-out angle φmin to the maximum lead-out angle φmax.

As described above, the guide surface 80 of the erecting base 74 is formed so that the treatment tool 56 led out of the conduit opening 86a of the treatment tool channel is bent and guided in a direction that becomes the intersection angle φ that can be determined in this way.

Next, advantages in a case where, as in the above present embodiment, the rotational axis Xa of the erecting base 74 is set to the direction of the horizontal axis X orthogonal to the scanning plane 102 and a treatment tool is guided and led out in the direction obliquely intersecting the scanning plane 102 by the guide surface 80 will be described.

As a form in which a treatment tool led out of the conduit opening 86a of the treatment tool channel is led out in the direction obliquely intersecting the scanning plane 102 of the ultrasonic transducer 60 by the erecting base, a form in which, as in JP2007-215634A and JP2010-4945A cited as prior art documents, a treatment tool is led out in the direction obliquely intersecting the scanning plane 102 by tilting the direction of the rotational axis of the erecting base obliquely from the direction orthogonal to the scanning plane 102 is considered in addition to the embodiment of the invention.

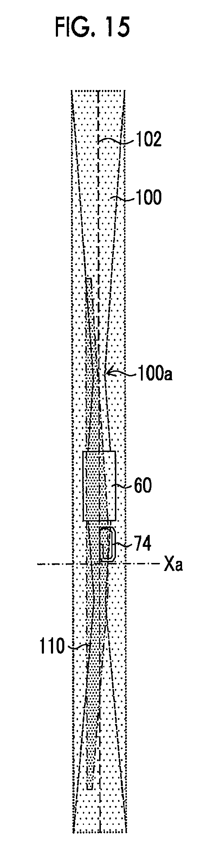

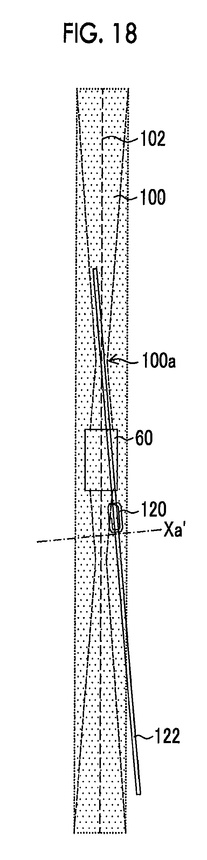

Fig. 18 is a plan view showing a relationship between a locus 122 drawn by the lead-out portion 56b of the treatment tool 56 when the erection angle of the erecting base 120 is changed, and the scanning region 100 in a case where the above form is adopted, and is a view corresponding to Fig. 15 in the embodiment of the invention. In addition, in Fig. 18 and the following description, the same constituent elements as those of the first embodiment are designated by the same reference numerals, and description thereof is omitted.

As shown in this drawing, the lead-out portion 56b of the treatment tool 56 is rotated around a rotational axis Xa' as the erecting base 120 is rotated around the rotational axis Xa' that is not orthogonal to the scanning plane 102. Therefore, the lead-out portion 56b of the treatment part 56 draws the locus 122 along a plane orthogonal to the rotational axis Xa'. In addition, the guide surface of the erecting base 74 is formed so as to guide the treatment tool 56 from the treatment tool channel in the direction (the direction of the central axis) orthogonal to the rotational axis Xa'.

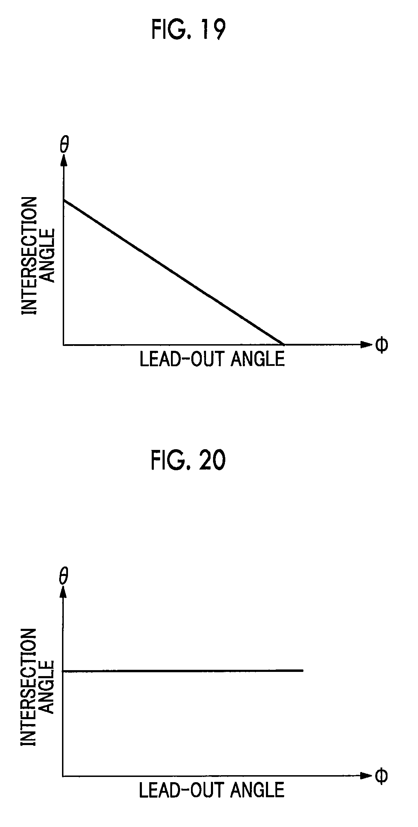

Accordingly, the intersection angle θ between the treatment tool 56 and the scanning plane 102 changes according to the erection angle of the erecting base 120, that is, according to the lead-out angle φ of the treatment tool 56, and as shown schematically on the graph of Fig. 19, the intersection angle θ becomes larger as the lead-out angle φ of the treatment tool 56 is smaller, and the intersection angle θ becomes smaller as the lead-out angle φ is larger. Therefore, it is difficult to satisfy the condition that the upper limit θmax of the allowable intersection angle range that allows the treatment tool 56 to pass through the scanning region 100 without deviating from the scanning region 100 as described above becomes smaller as the lead-out angle φ becomes smaller and the above upper limit becomes larger as the lead-out angle φ becomes larger, in a wide range of the lead-out angle φ, and the range of the lead-out angle φ where the treatment tool 56 can pass through the scanning region 100 without deviating from the scanning region 100 is restricted to an extremely narrow angle range.

On the other hand, as in the erecting base 74 of the embodiment of the invention, in a case where the rotational axis Xa of the erecting base 74 is set to the direction orthogonal to the scanning plane 102 and the treatment tool 56 is guided and led out in the direction intersecting the scanning plane 102 by the guide surface 80, as shown on the graph of Fig. 20, the intersection angle θ between the lead-out portion 56b of the treatment tool 56 and the scanning plane 102 can be kept constant in an arbitrary lead-out angle φ of the treatment tool 56. Accordingly, the treatment tool 56 can be configured so as to pass through the scanning region 100 without deviating from the scanning region 100 in a wide-range lead-out angle φ of the treatment tool 56.

By arranging the center position (central axis B) of the treatment tool lead-out portion 54 in the width direction so as to shift from the position on the same plane as the scanning plane 102 of the ultrasonic observation portion 50 (ultrasonic transducer 60) as described above, a wasteful non-used region of the tip rigid part 40 can be reduced, and the diameter of the tip rigid part 40 is reduced correspondingly. The invention is not limited to this case, and it is also possible to enlarge the treatment tool lead-out portion 54 without increasing the diameter of the present tip rigid part, thereby leading out a treatment tool channel with a larger diameter than the related art or adding other constituent elements. An embodiment in a case where a treatment tool lead-out portion is added as another constituent element will be described below.

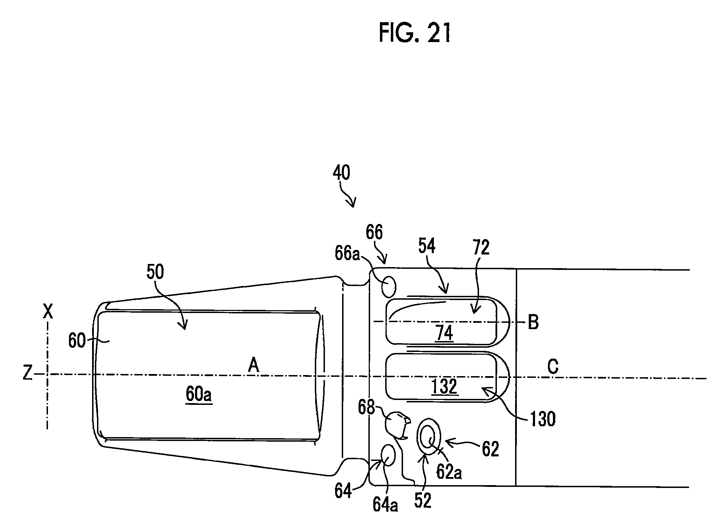

Fig. 21 shows a second embodiment in which two treatment tool lead-out portions (treatment tool lead-out ports) 54 and 130 are added to the tip rigid part 40. In addition, constituent elements with functions that are the same as or similar to those of the embodiment (first embodiment) shown in Figs. 3 to 6 or the like are designated by the same reference numerals, and description thereof is omitted.

As shown in this drawing, two treatment tool lead-out portions 54 and 130 are provided side by side at the tip rigid part 40, and the position (center position in the width direction) of a central axis C passing through the center of the width in the right-and-left direction (the direction of the horizontal axis X) in the treatment tool lead-out portion 130 (and erecting base 132) added in the present embodiment with respect to the first embodiment is arranged at a position on almost the same plane as the scanning plane 102 of the ultrasonic transducer 60.

Additionally, the erecting base 132 is rotated around the rotational axis orthogonal to the scanning plane 102 similar to the erecting base 74 of the treatment tool lead-out portion 54, and has a guide surface that guides and lead out a treatment tool from the treatment tool channel in the direction parallel to the scanning plane 102. In addition, since the configuration of the treatment tool lead-out portion 130 is the same as that of the treatment tool lead-out portion 54 described in the first embodiment except for the shape of the guide surface, detailed description thereof will be omitted.

The same observation optical system 62 (observation window 62a) and illumination optical system 64 (illumination window 64a) as those of the first embodiment are arranged in the region on the left from the base end side toward the tip side with respect to the treatment tool lead-out portion 130, and the same treatment tool lead-out portion 54 and illumination optical system 66 (illumination window 66a) as those of the first embodiment are arranged in the right region.

According to this, in the related art, one treatment tool lead-out portion 130 is included. In that case, another treatment tool lead-out portion 54 can be arranged effectively using a wasteful non-used region, and two treatment tool channels can be provided so that two treatment tools can be led out of the tip rigid part 40.

In addition, the treatment tool lead-out portion 130 may be arranged at a position where the center position (central axis C) in the width direction is shifted to any of right and left directions with respect to the scanning plane 102 similar to the treatment tool lead-out portion 54. In that case, similar to the erecting base 74 of the treatment tool lead-out portion 54 of the erecting base 132, the guide surface of the erecting base 132 may be formed so that a treatment tool from the treatment tool channel is guided and led out in the direction intersecting the scanning plane 102.

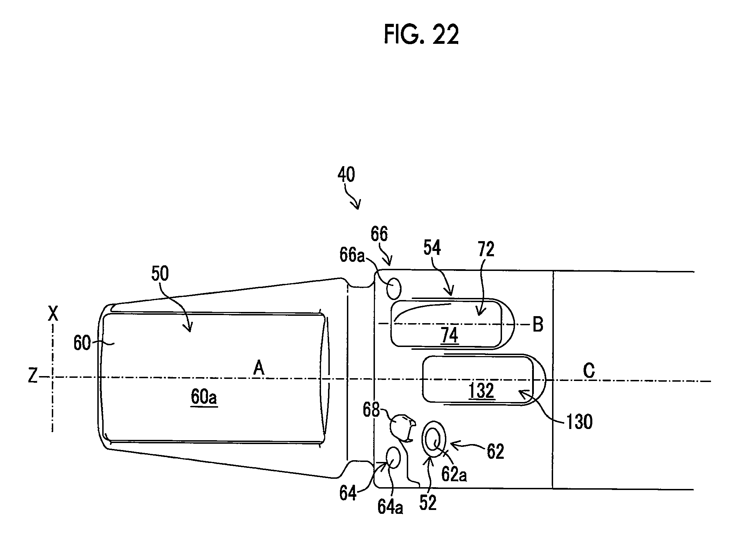

Additionally, if the positions of the two treatment tool lead-out portions 54 and 130 (erecting bases 74 and 132) in the direction of the insertion axis Z are shifted and arranged as shown in Fig. 22, since treatment tools led out of the respective treatment tool lead-out portions 54 and 130 enters an image from a position shifted on an ultrasonic image, it is possible to prevent a disadvantage that the treatment tools led out of the respective treatment tool lead-out portions 54 and 130 overlap each other on the ultrasonic image and cannot be identified. As shown in Fig. 22, the treatment tool lead-out portion 130 is not arranged further toward the base end side than the treatment tool lead-out portion 54, but the treatment tool lead-out portion 54 may be arranged further toward to the base end side than the treatment tool lead-out portion 130. Although a case where two treatment tool lead-out portions are provided side by side at the tip rigid part 40 has been described in the second embodiment shown in Figs. 21 and Fig. 22, the same configuration can also be applied to a case where three or more treatment tool lead-out portions are provided side by side.

As described above, in the above embodiment, the two illumination optical systems 64 and 66 that constitute endoscopic observation portion 52 are configured so as to be respectively arranged on both right and left sides of the treatment tool lead-out portion 54. However, since the illumination optical systems 64 and 66 do not occupy a large region as compared to the observation optical system 62, both of the illumination optical systems 64 and 66 may be arranged at any position, for example, may be arranged at a side portion on the side of the treatment tool lead-out portion 54 where the observation optical system 62 is arranged.

## Claims

1. An ultrasonic endoscope (10) comprising:
an insertion section (22) to be inserted into a body cavity;
an ultrasonic observation portion (50) located on a tip side (40) of the insertion section and having an ultrasonic transducer (60) that has a plane parallel to the longitudinal axis of the insertion section as a scanning plane;
a treatment tool lead-out port (54) arranged further toward a base end side than the ultrasonic observation portion, provided so as to be capable of leading out a treatment tool (56) inserted through a treatment tool channel of the insertion section, a central axis of the treatment tool lead-out port being arranged at a position apart in a normal direction from the scanning plane; and
an erecting base (74) arranged at the treatment tool lead-out port, configured so as to be rotatable around a rotational axis vertical to the scanning plane, changeably adjusting the erection angle of the treatment tool led out of the treatment tool lead-out port, and having a guide surface that bends the treatment tool led out of the treatment tool lead-out port in the direction of the scanning plane.

2. The ultrasonic endoscope according to Claim 1,
wherein the ultrasonic observation portion has a region having a predetermined width in a direction orthogonal to the scanning plane as a scanning region of an ultrasonic wave transmitted and received by the ultrasonic transducer, and
wherein the guide surface of the erecting base guides a lead-out portion of the treatment tool led out of the treatment tool lead-out port into the scanning region.

3. The ultrasonic endoscope according to Claim 1 or 2,
wherein an observation optical system is arranged at a side portion of the treatment tool lead-out port further toward the base end side than the ultrasonic observation portion.

4. The ultrasonic endoscope according to Claim 1, 2, or 3 comprising:
two erecting bases (74, 132) arranged at respective treatment tool lead-out ports (54, 130) of two treatment tool channels,
wherein at least one erecting base of the two erecting bases includes the guide surface.

5. The ultrasonic endoscope according to Claim 4,
wherein one erecting base of the two erecting bases is arranged further toward the base end side than the other erecting base.

## Patentansprüche

1. Ultraschallendoskop (2), umfassend:
einen Einführabschnitt (22) zum Einführen in einen Körperhohlraum;
einen Ultraschallbetrachtungsteil (50), angeordnet an einem Vorderende (40) des Einführabschnitts und ausgestaltet mit einem Ultraschallwandler (60), der als Abtastebene eine Ebene parallel zu der Längsachse des Einführabschnitts aufweist;
eine Behandlungswerkzeug-Ausleitöffnung (54), die weiter in Richtung eines Basisendes hin angeordnet ist als der Ultraschallbetrachtungsteil, und derart ausgebildet ist, dass sie in der Lage ist, ein Behandlungswerkzeug (56) auszuleiten, welches durch einen Behandlungswerkzeugkanal des Einführabschnitts eingeführt ist, wobei eine Mittelachse der Behandlungswerkzeug-Ausleitöffnung an einer Stelle angeordnet ist, die in einer normalen Richtung von der Abtastebene versetzt ist; und
eine Aufrichtbasis (74), angeordnet an der Behandlungswerkzeug-Ausleitöffnung, konfiguriert, um um eine Drehachse vertikal zu der Abtastebene drehbar zu sein, um den Aufrichtwinkel des aus der Behandlungswerkzeug-Ausleitöffnung ausgeleiteten Behandlungswerkzeugs veränderbar einzustellen, und ausgestattet mit einer Führungsfläche, welche das aus der Behandlungswerkzeug-Ausleitöffnung ausgeleitete Behandlungswerkzeug in die Richtung der Abtastebene biegt.

2. Ultraschallendoskop nach Anspruch 1,
bei dem der Ultraschallbetrachtungsteil eine Zone mit einer vorbestimmten Breite in einer Richtung orthogonal zu der Abtastebene als eine Abtastzone einer Ultraschallwelle aufweist, die von dem Ultraschallwandler gesendet und empfangen wird, und
wobei die Führungsfläche der Aufrichtbasis einen Ausleitabschnitt des aus der Behandlungswerkzeug-Ausleitöffnung ausgeleiteten Behandlungswerkzeugs in die Abtastzone führt.

3. Ultraschallendoskop nach Anspruch 1 oder 2,
bei dem ein optisches Betrachtungssystem an einem Seitenbereich der Behandlungswerkzeug-Ausleitöffnung weiter in Richtung des Basisendes als der Ultraschallbetrachtungsteil angeordnet ist.

4. Ultraschallendoskop nach Anspruch 1, 2 oder 3, umfassend:
zwei Aufrichtbasen (74, 132), die an jeweiligen Behandlungswerkzeug-Ausleitöffnungen (54, 130) zweier Behandlungswerkzeugkanäle angeordnet sind,
wobei mindestens eine Aufrichtbasis der beiden Aufrichtbasen die Führungsfläche enthält.

5. Ultraschallendoskop nach Anspruch 4, bei dem eine Aufrichtbasis der zwei Aufrichtbasen weiter in Richtung des Basisendes gelegen ist als die andere Aufrichtbasis.

## Revendications

1. Endoscope à ultrasons (10), comprenant :
une partie d'insertion (22) devant être insérée dans une cavité corporelle ;
une partie d'observation à ultrasons (50) située sur un côté formant bout (40) de la partie d'insertion et incluant un transducteur à ultrasons (60) présentant un plan parallèle à l'axe longitudinal de la partie d'insertion comme plan d'analyse ;
un orifice de sortie (54) pour outil de traitement agencé plus loin, vers un côté d'extrémité formant base, que la partie d'observation à ultrasons, prévu de manière à pouvoir faire sortir un outil de traitement (56) inséré à travers un canal pour outil de traitement de la partie d'insertion, un axe central de l'orifice de sortie pour outil de traitement étant agencé au niveau d'une position séparée dans une direction normale par rapport au plan d'analyse, et
une base de levage (74) agencée au niveau de l'orifice de sortie pour outil de traitement, configurée de manière à pouvoir tourner autour d'un axe de rotation verticalement au plan d'analyse, adaptant de manière variable l'angle de levage de l'outil de traitement sortant de l'orifice de sortie pour outil de traitement, et présentant une surface de guidage qui courbe l'outil de traitement sortant de l'orifice de sortie pour outil de traitement dans la direction du plan d'analyse.

2. Endoscope à ultrasons selon la revendication 1,
dans lequel la partie d'observation à ultrasons présente une région d'une largeur prédéterminée dans une direction orthogonale au plan d'analyse comme région d'analyse d'une onde ultrasonore transmise et reçue par le transducteur à ultrasons, et
dans lequel la surface de guidage de la base de levage guide une partie de sortie de l'outil de traitement sortant de l'orifice de sortie pour outil de traitement jusque dans la région d'analyse.

3. Endoscope à ultrasons selon la revendication 1 ou 2,
dans lequel un système optique d'observation est agencé au niveau d'une partie latérale de l'orifice de sortie pour outil de traitement plus loin, vers le côté d'extrémité formant base, que la partie d'observation à ultrasons.

4. Endoscope à ultrasons selon l'une quelconque des revendications 1 à 3, comprenant :
deux bases de levage (74, 132) agencées au niveau d'orifices de sortie pour outil de traitement respectifs (54, 130) de deux canaux pour outil de traitement,
dans lequel au moins une base de levage des deux bases de levage inclut la surface de guidage.

5. Endoscope à ultrasons selon la revendication 4,
dans lequel une base de levage parmi les deux bases de levage est agencée plus loin, vers le côté d'extrémité formant base, que l'autre base de levage.
